(19) Europäisches Patentamt
European Patent Office
Office européen des brevets

(11) Publication number: 0 317 226
A2

# (12) EUROPEAN PATENT APPLICATION

(21) Application number: 88310710.4

(22) Date of filing: 14.11.88

(51) Int. Cl.[4]: C07D 327/00 , C07D 321/00 , C07D 323/00 , C07D 493/08 , C08G 61/12 , C08G 75/00

(30) Priority: 17.11.87 GB 8726877

(43) Date of publication of application: 24.05.89 Bulletin 89/21

(84) Designated Contracting States: AT BE CH DE ES FR GB IT LI LU NL SE

(71) Applicant: IMPERIAL CHEMICAL INDUSTRIES PLC
Imperial Chemical House Millbank
London SW1P 3JF(GB)

(72) Inventor: Colquhoun, Howard Matthew
8 Grove Park
Knutsford Cheshire WA16 8QA(GB)
Inventor: Dudman, Christopher Curtis
9 Edwards Road
Leche Park Chester CH4 8HW(GB)
Inventor: Thomas, Mark
70 Rainbow Drive
Halewood Liverpool L26 7AG(GB)

(74) Representative: Chapman, Kenneth Hazel et al
Imperial Chemical Industries PLC Legal Department: Patents P.O. Box 6 Bessemer Road
Welwyn Garden City Herts, AL71HD(GB)

## (54) Aromatic compounds.

(57) A macrocyclic compound contains linked together in a macrocycle at least one polyarylene unit containing 2 or more arylene rings and at least 2 other cyclic units, each of said polyarylene unit and said other units being linked to its neighbouring unit by a divalent single atom group or by an alkylene group. The compound can be made by ring-closure of a linear compound containing the arylene rings and other cyclic units and having 2 terminal halogens, preferably by the coupling reaction by zerovalent nickel. The compound can be polymerised by ring-opening in the presence of a catalytic quantity of a nucleophile.

Fig.1b.

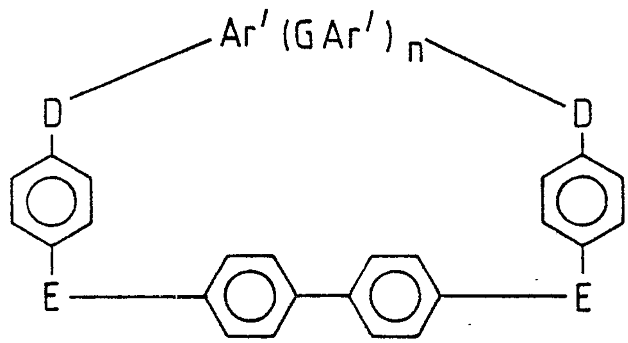

EP 0 317 226 A2

Xerox Copy Centre

## Aromatic compounds

This invention relates to aromatic compounds, in particular to macrocyclic compounds ("cyclomers") containing aromatic rings and ether and/or thioether linkages and to a polymerisation process and polymers based on such compounds.

The invention provides in its first aspect a macrocyclic compoundin which there are linked together in a macrocycle at least one polyarylene unit containing 2 or more arylene rings and at least 2 other cyclic units, each of said polyarylene unit and said other units being linked to its neighbouring unit by a divalent single atom group as hereinafter defined or by an alkylene group.

The divalent single atom group is a divalent atom or a molecular group having 2 available valencies on the same atom. It can be for example any of NR, O, S, CO, $SO_2$, P(R), PO(R), or aliphatic hydrocarbon or fluorocarbon where R is a hydrocarbon group typically containing up to 6 carbon atoms. If it is aliphatic hydrocarbon or fluorocarbon, then the rings are linked to the same carbon atom and that carbon atom carries preferably two hydrocarbon or fluorocarbon groups, especially methyl or trifluoromethyl, other lower alkyl (up to $C_6$), ring-forming alkylene (up to $C_6$) or aryl. If the link is through alkylene, that group contains preferably 2 to 6 carbon atoms. In a given macrocyclic compound divalent groups of more than one type can be present. If desired, there may be, additionally to a divalent single atom group, a direct link or a second single atom group between any two neighbouring aromatic hydrocarbon units, thus giving a fused ring system unit, but usually the number of aromatic rings in such units so linked is not more than one third of the total aromatic rings (including the arylenes of the polyarylene) in a given macrocyclic compound. Examples of such fused ring system units are dibenzofuran, dibenzothiophene, dibenzothiophene dioxide, fluorene, fluorenone, xanthone, napthalene, anthraquinone, phenanthrene and bisphthalimide.

In the accompanying drawings:

Figure 1 shows 4 general formulae a-d of compounds according to the invention;

Figure 2 shows the spatial structure of the compounds described in the Examples.

The macrocyclic compound can be as represented by formula a in figure 1 which each pair of adjacent groups Ar constitutes a said polyarylene unit, m is 0 or 1, J contains at least 2 cyclic units, and each linkage between the cyclic units within J and between J and Ar is a single atom group as hereinbefore defined or an alkylene group. Ar is arylene, especially phenylene, bisphenylene or naphthalene, possibly carrying one or more substituents as detailed below.

In preferred compounds the polyarylene unit constituted by the groups Ar contains an even number of arylene units and is especially 4,4′-biphenylene. The number of said units in the ring is preferably at least 2.

The compound preferably contains within J the succession

D - Ar′ - E

where Ar′ is an aromatic ring capable of transmitting the nucleophilic activating effect of an electron-withdrawing group and optionally carries one or more inert substituents, D is O or S, E is an electron-withdrawing group and the linkages of D and E to Ar′ are such that D is activated by E. More preferably the succession is

E - Ar′ - D - Ar′ - E

In any such compound there may be a plurality of different groups Ar′, D or E. Either such succession may occur more than once, with 2 such successions possibly sharing a common group E. Examples of inert substituents on Ar′ are given below.

A preferred macrocyclic compound has the general formula 1b in the accompanying drawing where Ar′, D and E are as defined above, n is 0, 1 or 2 and G is a direct link or a single atom group or alkylene group as defined above. As in the above broader definition, in any ring groups D may be the same or different and the same applies to E and Ar′. The group Ar′ (G Ar′)$_n$ can thus be a single aromatic ring for example phenylene or one of the rings of a fused ring system such as naphthalene or anthracene. If it contains 2 or more aromatic rings linked together by group G, then group G is preferably an electron withdrawing group, especially $SO_2$ or CO. There may be more than one group G between 2 groups Ar′, thus giving a fused ring system.

Examples of Ar′ (G Ar′)$_n$ are:

phenylene and polyphenylenes

diphenyl ether or thioether and higher ethers and thioethers such as bisphenoxybenzene or bisphenylthiobenzene

diphenylsulphone
benzophenone
naphthalene, anthracene or phenanthrene
diphenylmethane
2:2 -diphenylpropane
dibenzofuran
xanthone
bis phthalimido phenylene.

The linkages in the grouping D Ar′ (G Ar′)$_n$D are preferably mutually para or meta or mixed para and meta. Preferably D is para to G at each occurrence, especially if G is an electron withdrawing group.

The invention provides a process for making the macrocyclic compound by reacting a halogen compound of general formula

X - Ar - J - Ar - X

where X is halogen, other than fluorine; and

Ar and J are as hereinbefore defined;

with a metal effective to form a halide and unite the said groups Ar to form the said polyarylene unit. In any such starting halogen compound the groups Ar can be the same or different.

Certain examples of the starting halogen compound are believed to be new, in particular those of general formula

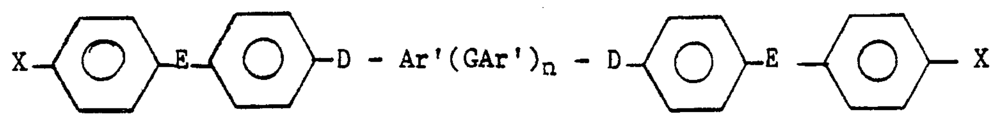

where Ar′, D, E, G and n are as herein before defined.

The substituents on groups Ar are preferably as follows:

a) if J provides a link to Ar through an electron withdrawing group such as $SO_2$, SO, CO, P(O)R, positioned so as to activate X, then the substituents can be any that are inert in the reaction, for example hydrocarbon, hydrocarbonoxy or hydrocarbonthio or fluorine; or the positions not occupied by X and J can be all hydrogen;

b) if J provides a link to Ar otherwise than through an electron withdrawing group, then Ar carries such a group, for example CN, $CF_3$, acyl or hydrocarbonsulphonyl in a position meta or para with respect to X and ortho with respect to the link to J. There may be other substituents as listed in (a);

c) the positions ortho to X are occupied by hydrogen.

The above-mentioned inert substituents are those possibly present on Ar′.

The metal is very suitably from the iron group, especially nickel. Conveniently it is in the ligand-stabilised zerovalent state, such as is provided by complexing a nickel salt, especially a halide, with a suitable ligand, especially a triaryl phosphine, for example triphenyl phosphine, and reducing the nickel to the zerovalent state, suitably by means of a more electropositive metal such as magnesium, manganese or zinc or more than one of these. Conveniently the reaction of the halogen compound with the zerovalent nickel is carried out in the presence of electropositive metal in excess with respect to the halogen compound. The quantity of zerovalent nickel is preferably at least stoichiometrically equivalent to the halogen X to be reacted, but can be less since it is continuously re-reduced by the more electropositive metal. Over-all, the reaction of the halogen compound is with the more electropositive metal and the action of the complexed nickel compound is essentially that of a catalyst.

The reaction of the halogen compound with the metal is carried out typically as a temperature in the range 10 - 100 °C in a polar aprotic solvent such as dialkylamide, and preferably at a low concentration of that compound, so as to decrease the chance that two groups X will be taken from two different molecules (thus forming a linear compound or larger ring) and thus to form preferentially the macrocyclic compound having m = 0 in formula 1. Such low concentration can be obtained by the dropwise addition of a solution of the halogen compound into a solution containing the zerovalent nickel and the electropositive metal. The solution to be added contains the compound in the concentration range typically 0.1% to 10% w/v. If desired, however, the reaction can be controlled so as to form a substantial quantity of a macrocyclic compound in which two of the residues Ar-J-Ar are present in the same macrocycle (that is m = 1 in figure 1a); such residues can be the same or different.

Alternative processes for uniting the groups Ar are, for example the Ullmann reaction over metallic

copper.

The invention provides in its second aspect a process for producing a polymer containing the repeating unit

- D - Ar′ - E - Ar′ -

which comprises ring-opening a macrocyclic compound containing that unit, where D and E are as hereinbefore defined, Ar′ is an aromatic ring capable of transmitting the nucleophilic activating effect of an electron withdrawing group, and the position of E with respect to D is such that D is so activated by E. In such a polymer the groups D, E and Ar′ may respectively be the same or different.

More specifically the macrocyclic compounds have the formulae as shown in the accompanying drawing. Starting from formula 1b the polymer contains and preferably consists essentially of the repeating unit

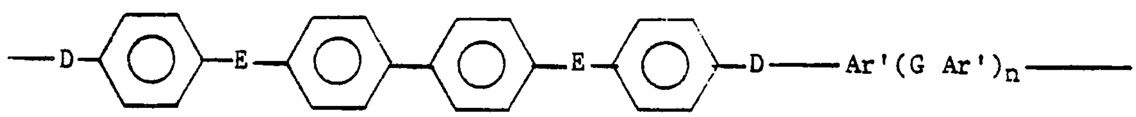

Thus in a polymer formed from compound 1C the repeating unit is

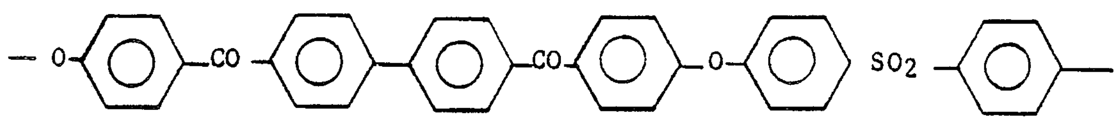

and the repeating units in polymers formed from compounds 1d can be readily envisaged. Ring opening can be effected by any nucleophilic reagent that will cleave an aromatic ether or thioether to produce one nucleophilic end group. Usually this is an alkali metal compound, especially of K, Rb or Cs. The anion of the reagent is suitably fluoride, phenoxide, thiophenate, alkoxide or alkylmercaptide. The nucleophilic reagent should be present in a catalytic quantity, which typically is in the range 0.01 to 10% molar on the starting macrocyclic compound.

In the polymerisation the molecular weight can if desired be limited by end-capping, for example by means of an alkyl halide or, more conveniently by means of an activated aryl halide such as one of general formula -E-Ph-X′, where X′ is halogen. A suitable end-capping agent is a (di) halogeno-benzophenone or -diphenylsulphone, particularly (in view of its low volatility) 4-benzoyl-4′-(4-fluorobenzoyl)-biphenyl.

Formation of polymers by the process of the invention has one or more of the following advantages, as compared with conventional nucleophilic or electrophilic polycondensation:

a) The polymerisation takes place in the absence of solvent so no leaching or other workup is necessary.

b) The reaction is very rapid, being possibly essentially complete within 1 - 10 minutes depending on the amount of catalyst present.

c) No by-products are formed, giving polymer of low impurity content.

d) The reaction melt is initially of low viscosity enabling the manufacture of finely detailed articles by in situ polymerisation. In addition, the melt flows easily over surfaces giving good adhesion for a protective coat or as an adhesive.

e) The succession of units in the polymer chain can be fixed and reproducible, giving highly reproducible polymer characteristics, unless polymerisation conditions are changed so as to cause randomisation of the units within the chain.

f) Only a single reactant monomer need be used, fixing the stoichiometry of the polymer and further enhancing reproducibility of polymer characteristics.

The polymers can be prepared with molecular weights corresponding to inherent viscosities typically above 0.2, especially in the range 0.3 to 1.5, possibly so high as to be difficultly soluble in sulphuric acid S.G.1.84.

The invention provides further a method of making a shaped article by confining a quantity of a macrocyclic compound according to the invention of a low polymer thereof and polymerising it. Examples of the method are:

extrusion of fibre or film as tube or wire coating, in which passage through a shaping zone is followed by

passage through a polymerisation zone;
casting, compression moulding or injection moulding;
coating a shaped article;
adhesively uniting shaped articles;
impregnating fibres and/or uniting plies of impregnated fibres.

Example 1

a) Preparation of 4,4′-diphenoxydiphenylsulphone

Phenol (20 g 0.22 mole) and 4,4′-dichlorodiphenylsulphone (28 g 0.1 mole) were dissolved in a mixture of toluene (500 ml) and N,N dimethylacetamide (500 ml). Anhydrous potassium carbonate (28 g 0.2 mole) was added and the mixture was brought to reflux under nitrogen. The water produced was removed with a Dean-Stark apparatus: when all the water had been removed, the toluene was distilled out until the internal temperature reached 150 - 160 °C. The mixture was refluxed for four hours, then allowed to cool and poured into water (1000 ml). The solid which precipitated was recovered by filtration, washed with water, dried and recrystallised from toluene or chlorobenzene. Yield: 32 g 80%. Melting point: 140-2 °C.

b) Preparation of 4,4′-di-(4-(4-chlorobenzoyl)phenoxy)diphenylsulphone

Anhydrous aluminium chloride (5.33 g .04 mole) was suspended in 1,2-dichlorobenzene (50 ml) under nitrogen and 4-chlorobenzoylchloride (4 g .0228 mole) was added. To this solution was added 4,4′-diphenoxydiphenylsulphone (4 g 0.01 mole) and the whole was heated to 40 °C for 2 hours, then at 100 °C for a further 2 hours. The mixture was then filtered and poured into ice-water (200 ml) containing concentrated hydrochloric acid (15 ml). The precipitate was recovered by filtration and washed with water, then dried and recrystallised from toluene. Yield: 5.36 g 70%. Melting point: 225-7 °C.

c) Cyclisation

To 800 ml of dry N,N-dimethylacetamide in a dry 3-litre flask under nitrogen was added triphenylphosphine (38 g 0.145 mole), tetra-N-butylammonium iodide (27 g 0.073 mole) and anhydrous nickel chloride (2.4 g 0.185 mole). The mixture was stirred for 10 minutes, then finely powdered zinc (13.2 g 0.2 moles) was added and the mixture was heated to 60 °C and stirred for a further 30 minutes. A solution of 4,4′-di-(4-(4-chlorobenzoyl)phenoxy)diphenylsulphone (14 g 0.021 mole) in dry N,N-dimethylacetamide was added dropwise with stirring over 5 hours. After the addition was complete, the mixture was stirred at 60 °C for a further 30 minutes, then cooled and filtered to remove unreacted zinc. The filtrate was poured into water (2.5 l) and the precipitate was collected by filtration, washed with aqueous methanol and dried. The solid was then stirred in 100 - 120 °C petroleum ether (500 ml) at 80 °C and the suspension was filtered hot. The solid was dried and recrystallised from chlorobenzene to give the cyclomer III (Y = H) which was shown to be pure by HPLC. Yield: 3 g 24%. Melting point: 353-5 °C.

Mass spectrometory showed a strong parent ion ($M_+$) at m/e 608, and infra-red spectrometry (hydrocarbon mull) showed a strong carbonyl stretching absorption at 1670 $cm^{-1}$, a rather high frequency for a diaryl ketone and consistent with the carbonyl group being located in a strained macrocyclic ring. $^{13}C$ NMR spectrometry in dimethylsulphoxide solution showed thirteen separate resonances, consistent with the expected presence of thirteen chemically distinguishable carbon atoms in the proposed cyclic structure. Crystals grown from toluene solution contained 1 mole of toluene per mole of cyclomer, and single crystal X-ray analysis of this material revealed the cyclomer structure shown in Figure 2a.

**Crystal data:**

MW + 700.8, monoclinic, space group $P2_1/n$, $\underline{a}$ = 11.403(2), $\underline{b}$ = 18.815(4), $\underline{c}$ = 17.273(4) Å,$\alpha$ = 106.63(1)°, $\underline{U}$ = 3551 Å$^3$, $\underline{Z}$ = 4, R = 0.044.

Example 2

Stage (a) of Example 1 was repeated. Stage (b) of Example 1 was repeated, with the exception that 4-chloro-2-fluorobenzoyl chloride (4,42 g 0.0228 mole) was used in the place of the 4-chlorobenzoyl-chloride. The product was obtained in 80% yield, melting point 251 °C. Stage (c) was carried out in the same way giving cyclomer 1c (Y = F) in 35% yield, melting point 383 °C.

Mass spectrometory showed a strong parent ion (M+) at m/e 644, and infra-red spectrometry (hydrocarbon mull) showed a strong carbonyl stretching absorption at 1680 $cm^{-1}$. $^{13}C$ NMR spectrometry in dimethylsulphoxide solution showed fifteen resonances (six of which were split by $^{19}F$-$^{13}C$ coupling) expected for the proposed cyclic structure.

Example 3

a) Preparation of 3,6-diphenoxydibenzofuran

Phenol (270 g 3 moles) was dissolved in toluene (120 ml) and potassium hydroxide (67.2 g 1.2 moles) was added. The mixture was refluxed under nitrogen, the water being removed using a Dean-Stark trap. The temperature was then raised, and the toluene was distilled off. When all the toluene had been removed, anhydrous cupric chloride (6 g 0.04 moles) and 3,6-dibromodibenzofuran (48 g 0.147 moles) was added. The temperature was raised to 190 °C and the mixture was stirred overnight. The mixture was then cooled and added to 3 M potassium hydroxide solution (3 litres). The organic material was extracted into toluene (4x500 ml), and the toluene solution was washed with potassium hydroxide solution, water, EDTA solution until all the copper had been extracted, and water. The toluene solution was dried and evaporated to give a white solid which was recrystallised from methylated spirit/toluene. Yield: 33.5 g 65%. Melting point: 120 °C.

b) Preparation of 3,6-di(4-(4-chlorobenzoyl)phenoxy)dibenzofuran

The 3,6-diphenoxydibenzofuran was reacted with 4-chlorobenzoyl chloride as in Example 1(b). The product was obtained in 69% yield and melts at 200 °C.

c) Cyclisation

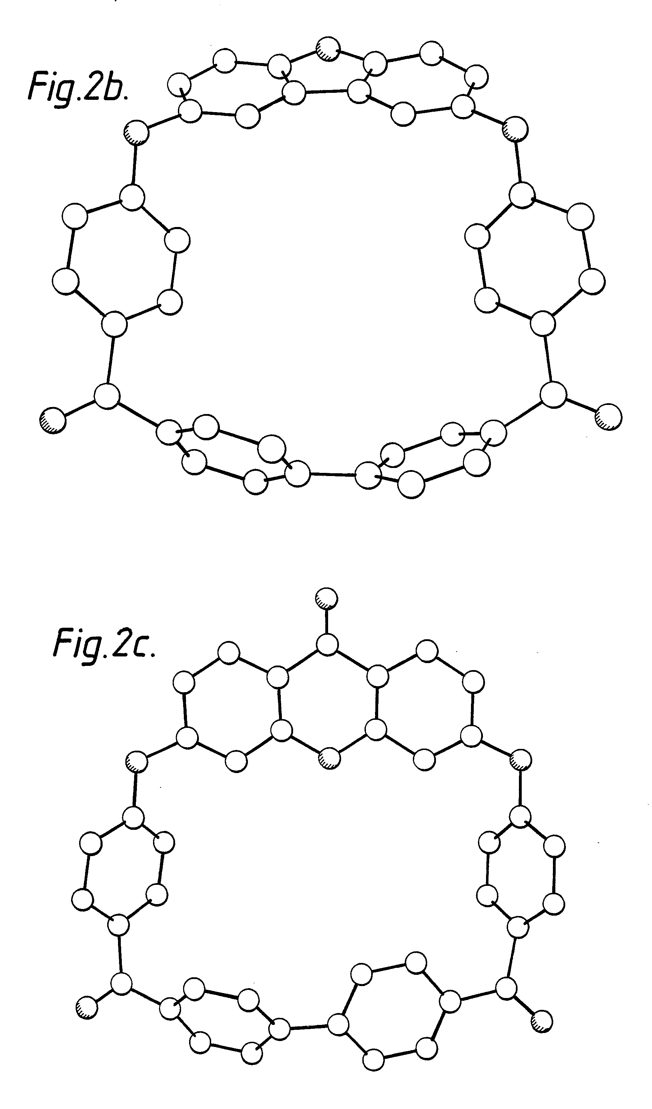

The product from Example 3(c) was cyclised in the manner of Example 1(c) to cyclomer 1d (T is direct link) in 40% yield with melting point 353 °C.
Mass spectrometry showed a strong parent ion at m/e 558, and $^{13}C$ NMR spectrometry showed the expected fifteen resonances, although several of these were split as a result of sterically restricted rotation of the oxybenzoyl groups within the macrocyclic structure. Single crystals grown from toluene solution contained 0.5 moles of toluene per mole of cyclomer, and X-ray analysis of one such crystal revealed the cyclomer structure shown in Figure 2b.

**Crystal data:**

MW = 604.6, triclinic, space group P1, $\underline{a}$ = 9.962(1), $\underline{b}$ = 12.777(2), $\underline{c}$ = 13.369(2) Å, $\alpha$ = 69.12(1), $\beta$ = 73.45(1), $\gamma$ = 89.13(1)°, $\underline{U}$ = 3551 Å$^3$, $\underline{Z}$ = 2, R = 0.048.
Strain Energy: The enthalpy of combustion of the pure, solvent-free cyclomer shown in Figure 2b was measured as 17903.8 kJ $mol^{-1}$. Comparison with the value for an open-chain analogue gave a figure for the ring-strain-energy of 200 ± 5 kJ $mol^{-1}$. This value is in keeping with the pronounced tendency of the present family of cyclomeric compounds to undergo ring-opening polymerisation in the presence of nucleophilic initiators.

Example 4

a) Preparation of 3,6-di-(4-(4-chlorobenzoyl)phenoxy)xanthone

3,6-dihydroxyxanthone (9.2 g 0.04 mole) and 4-chloro-4′-fluorobenzophenone (23.4 g 0.1 mole) were dissolved in a mixture of toluene (500 ml) and N,N-dimethylacetamide. Anhydrous potassium carbonate (5.44 g 0.04 mole) was added and the mixture was refluxed with stirring under nitrogen. The water was removed using a Dean-Stark apparatus, and when all the water had been removed the toluene was distilled out. The mixture was heated to 170°C for 4 hours then cooled and poured into water (1000 ml). The precipitate was recovered by filtration, washed with water and methylated spirit, then dried and recrystallised from chlorobenzene to give the product in 82% yield, melting point 226°C.

b) The product obtained in Example 4(a) was cyclised by the procedure outlined in Example 1(c) to give cyclomer 1d (T is CO), yield 21%, melting point 410°C.

Mass spectrometry showed a strong parent ion ($M^+$) at m/e = 586. Single crystals grown from toluene were unsolvated, and an X-ray diffraction study revealed the cyclomer structure shown in Figure 2c.

**Crystal data:**

MW = 586.6, triclinic, space group P $\bar{1}$, a = 10.567(3), b = 15.223(4), c = 20.307(7) Å, α = 99.62(3), β = 103.81(2), γ = 107.69(2)° Å³, Z = 4 (two independent molecules per unit cell), R = 0.109.

Example 5

Polymerisation of Cyclomers

A 1 mM solution of caesium fluoride in anhydrous methanol was prepared, and 0.5 g of cyclomer 1c (Y = H) was suspended in 100 ml of this solution. This corresponds to 1.6 mole % of fluoride on cyclomer. The sample was dried and divided into 50 mg portions, each of which was treated separately.

a) The sample was pressed into a pellet and heated in a differential scanning calorimeter (DSC) under nitrogen. At the melting point (353°C) an exotherm was seen, corresponding to ring opening polymerisation taking place. After heating for five minutes at 370°C, the sample was cooled to 50°C and scanned to 400°C. No melting peak was observed, instead a Tg was seen at 210°C indicating that polymerisation had gone to completion, giving high molecular weight polymer. The polymer was difficultly soluble in concentrated sulphuric acid.

b) The sample was mixed thoroughly with 4.8 mg of 4-benzoyl-4′-(4-fluorobenzoyl)biphenyl as end-capper then treated as in (a). Again polymerisation was complete, the polymer having Tg 197°C indicating a lower molecular weight than that obtained in (a). The polymer was soluble in sulphuric acid S.G.1.84 and had inherent viscosity 0.64 as determined at 25°C using 0.1 g polymer in 100 ml of solution.

## Claims

1. A macrocyclic compound in which there are linked together in a macrocycle at least one polyarylene unit containing 2 or more arylene rings and at least 2 other cyclic units, each of said polyarylene unit and said other units being linked to its neighbouring unit by a divalent single atom group or by an alkylene group containing 2 to 6 carbon atoms.

2. A macrocyclic compound to claim 1 having the general formula

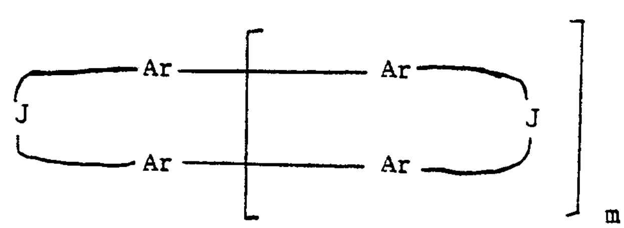

in which Ar is arylene, each pair of adjacent groups Ar constitutes a said polyarylene unit. m is 0 or 1, and J contains the said at least 2 cyclic units in the succession

- E - Ar' - D -

where Ar' is an aromatic ring capable of transmitting the nucleophilic activating effect of an electron-withdrawing group;

D is O or S;

E is an electron-withdrawing group; and

the linkages of D and E to Ar' are such that D is activated by E.

3. A macrocyclic compound according to claim 1 or claim 2 and having the formula of figure 1b in the accompanying drawing, where

n is 0, 1 or 2; and

G is a direct link or a single atom group or alkylene group containing 2 to 6 atoms.

4. A process for making macrocyclic compound according to any of claims 1 to 3 by reacting a halogen compound of general formula

X - Ar - J - Ar - X

where X is halogen, other than fluorine and Ar and I are as defined in claim 2 with a metal effective to form a halide and unite the said groups Ar to form the said polyarylene unit.

5. A process according to claim 4 in which the halogen compound is reacted with zerovalent nickel stabilised by a triarylphosphine in a polar aprotic solvent in the presence of a more electropositive metal selected from magnesium, manganese or zinc in excess with respect to the halogen compound.

6. A process according to claim 5 in which a solution of the halogen compound is added dropwise into a solution containing the zerovalent nickel and the electropositive metal, whereby to form preferentially a macrocyclic compound according to formula 1a in which m = o.

7. A process for producing a polymer containing a repeating unit.

- D - Ar' - E - Ar' -

which comprises ring-opening a macrocyclic compound containing that unit, where D is O or S, E is an electron-withdrawing group, Ar' is an aromatic ring capable of transmitting the nucleophilic activating effect of an electron withdrawing group, and the position of E with respect to D is such that D is so activated by E.

8. A process according to claim 7 in which ring-opening is effected by a fluoride, phenoxide, thiophenate, alkoxide or alkylmercaptide of potassium, rubidium or caesium in a concentration of 0.01 to 10% molar on the starting macrocyclic compound.

9. A compound of general formula

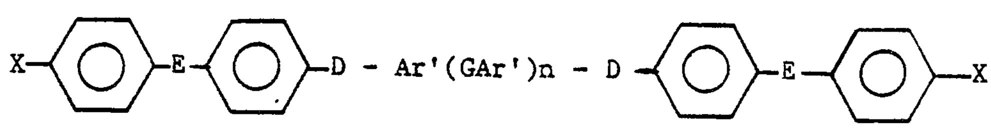

where Ar' is an aromatic ring capable of transmitting the activating effect of an electron-withdrawing group;

D is O or S;

E is an electron-withdrawing group;

G is a direct link or a single atom group or an alkylene group containing up to 6 carbon atoms;

n is 0, 1 or 2; and

X is halogen other than fluorine.

10. A method of making a shaped article by confining a quantity of of a macrocyclic compound according to any one of claims 1 to 3 or a low polymer thereof and polymerising it by a process according to claim 7 or 8.

Fig.1a.

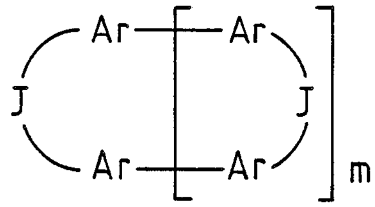

Fig.1b.

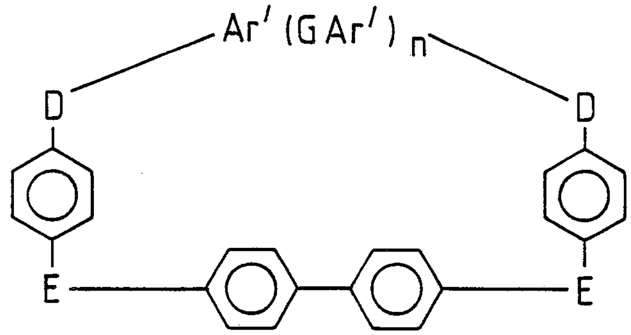

Fig.1c.

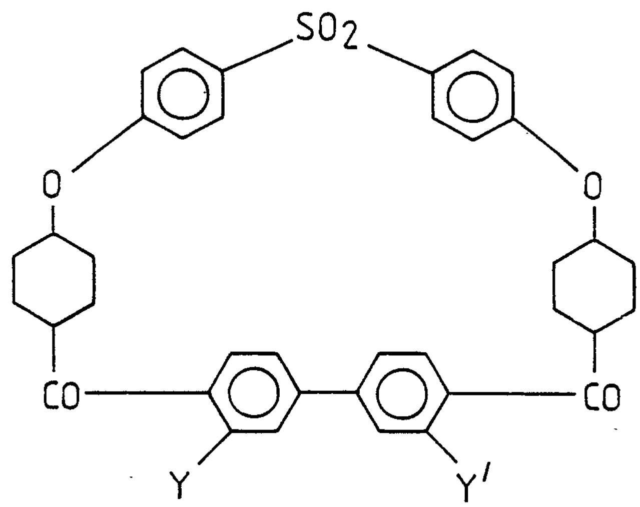

Fig.1d.

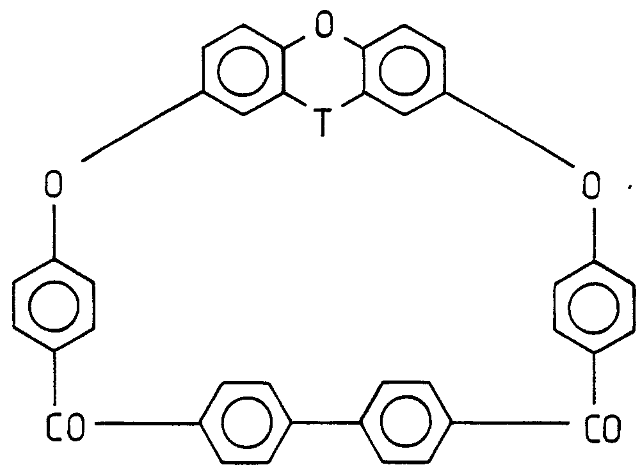

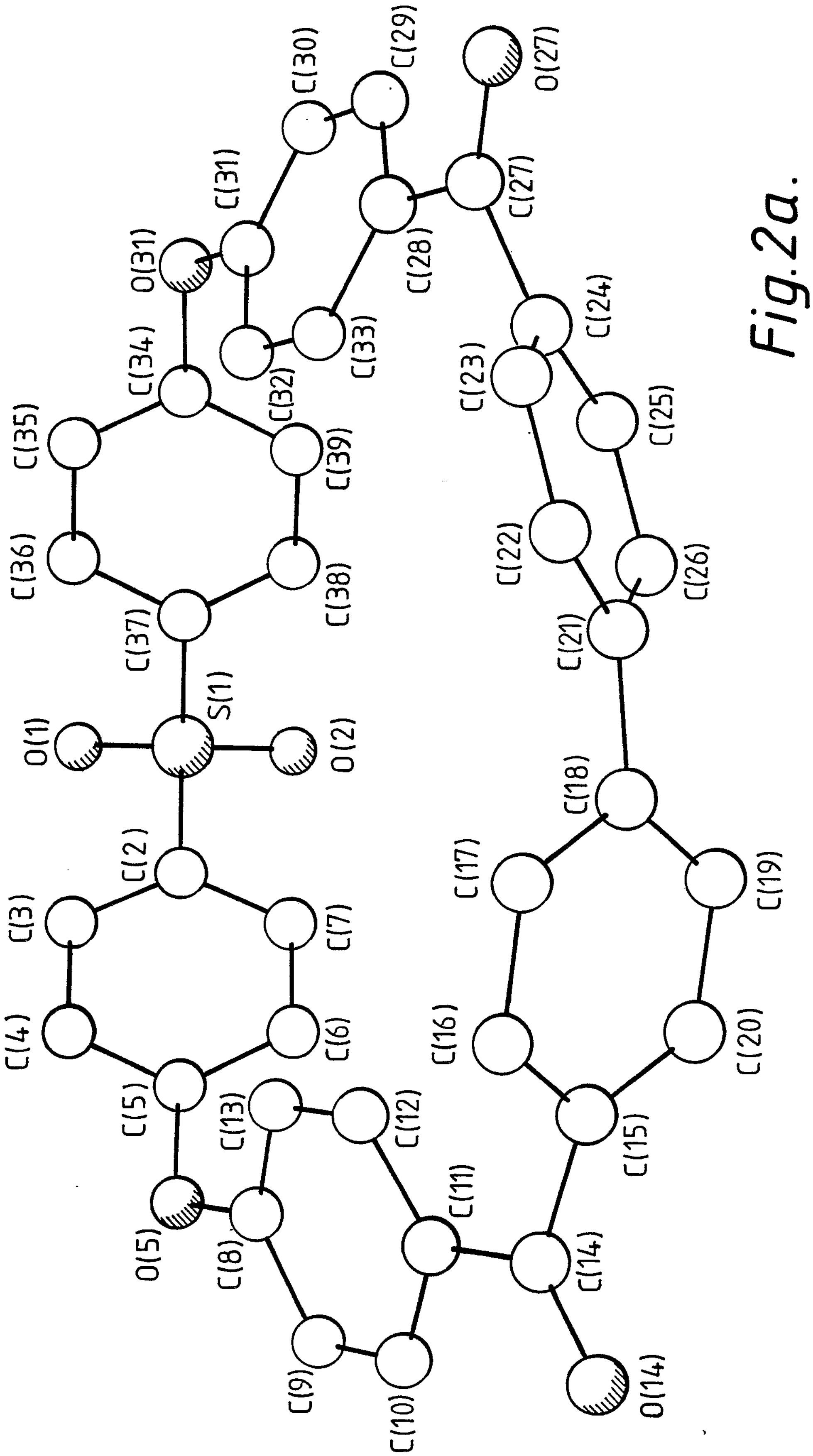

Fig.2a.

Fig.2b.

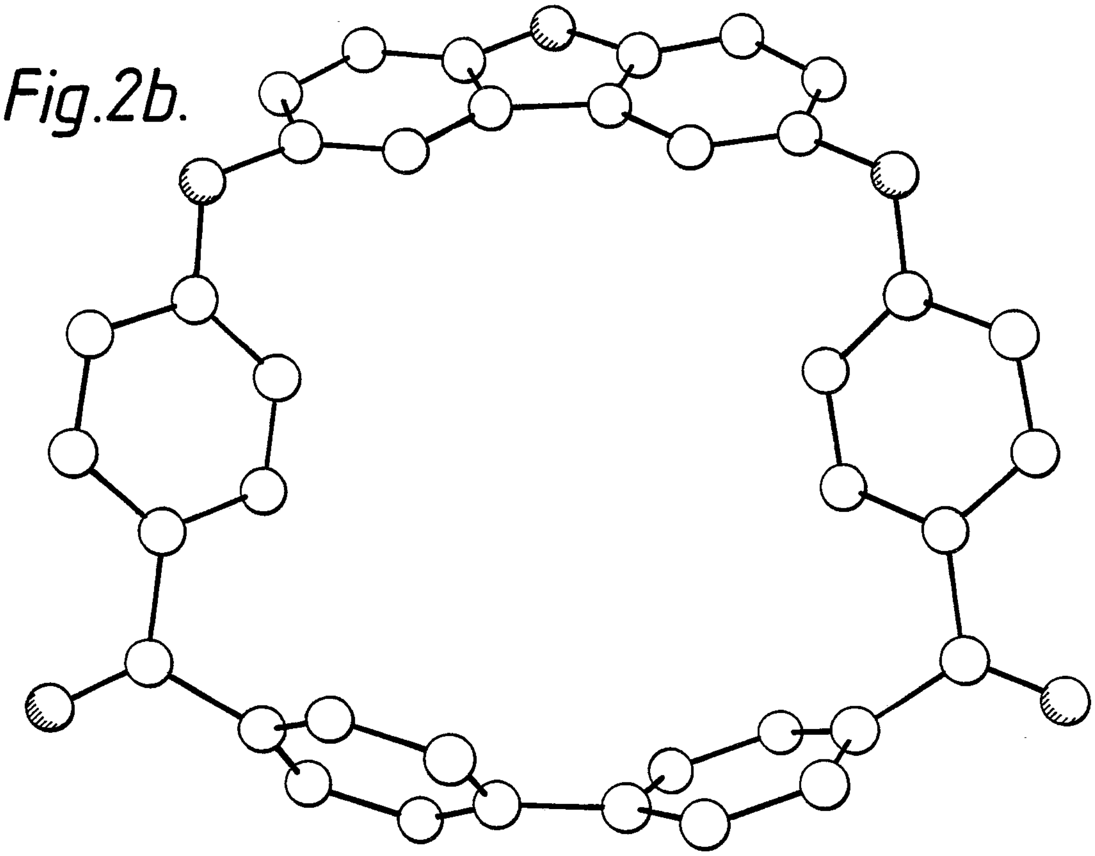

Fig.2c.